# EUROPEAN PATENT APPLICATION

(11) **EP 3 777 567 A1**
(43) Date of publication of application: **17.02.2021**
(21) Application number: 19774759.5
(22) Date of filing: 27.03.2019
(51) Int. Cl.: A23L 33/19, C07K 14/47

(54) **METHOD FOR PRODUCING COMPOSITION CONTAINING k-CASEIN GLYCOMACROPEPTIDE**

(30) Priority: 30.03.2018 JP 2018067061; 30.03.2018 JP 2018067059; 14.12.2018 JP 2018234335
(71) Applicant: Megmilk Snow Brand Co. Ltd., Sapporo-shi, Hokkaido 065-0043 (JP)
(72) Inventor: TAMAKI Shojiro, Sapporo-shi, Hokkaido 065-0043 (JP); ITOH Daisuke, Sapporo-shi, Hokkaido 065-0043 (JP); FUKUDOME Hirofumi, Sapporo-shi, Hokkaido 065-0043 (JP); NAKANO Taku, Sapporo-shi, Hokkaido 065-0043 (JP)
(74) Representative: Potter Clarkson
(86) International application number: PCT/JP2019/013166
(87) International publication number: WO 2019/189350

(57) **Abstract**

The present invention relates to a method for producing a composition containing κ-casein glycomacropeptide including: (A) preparing an aqueous solution containing a milk ingredient having a pH of 4 to 9; (B) pretreating the aqueous solution to obtain an aqueous solution containing carbonate (hydrogen) ions and metal ions; (C) subjecting the pretreated aqueous solution to membrane treatment using a membrane having a molecular weight fraction of 9,000Da or more and 300,000Da or less. According to the method, it is possible to obtain a composition containing GMP, which is simple to operate and has a high yield regardless of whether the starting material is cheese whey or whey protein concentrate.

## Description

### TECHNICAL FIELD

The present invention relates to a method for producing a composition containg κ-casein glycomacropeptide (hereinafter also referred to as "GMP") . The present invention also relates to a method for producing a composition containg GMP and α-lactalbumin (hereinafter also referred to as "LA") .

### BACKGROUND ART

GMP, which is one of sialic acids contained in milk, is also called caseinomacropeptide (hereinafter also referred to as "CMP"), and is a sialic acid-binding peptide produced when rennet or pepsin is applied to κ-casein of milk. GMP is conventionally known to be included in cheese whey and rennet casein whey. It is said that the molecular weight of GMP is about 9,000 Da, which present as monomers at pH of less than 4 and polymers (molecular weight 50,000Da or more) at pH of 4 or more.

Breast milk contains about 3 to 5 times more sialic acids than milk, and this sialic acid is also thought to function as one of the protective factors against infection in infants. GMP, one of the sialic acids, is also known to have protective action against viruses and bacteria and lactic acid bacteria proliferative activity. LA, like GMP, is a major protein that accounts for about 30% of the proteins contained in breast milk. LA, like GMP, is known to have various bioactive functions such as protection of gastric mucosa. LA is a protein with a molecular weight of about 14,000Da.

Therefore, there is a strong demand for industrial-scale production of GMP, which is a sialic acid, as a food product such as a milk substitute and a functional food. As a technique for fractionating GMP which is sialic acid in milk, there has been cited a method using ultrafiltration, a reverse osmosis membrane, or an ion exchange resin.

Patent Document 1 discloses a method for producing GMP characterized by (1) adjusting a GMP-containing milky raw material to a pH value less than 4; then performing ultrafiltration membrane treatment of a fractional molecular weight of 10,000 to 50,000Da to obtain a permeate; and concentrating the permeate by using a membrane of a fractional molecular weight of 50,000Da or less. Patent Document 1 discloses a method for obtaining a composition having a high GMP content by concentrating the permeate obtained in (1) using an ultrafiltration membrane having a fractional molecular weight of 50,000Da or less after the permeate obtained in (1) is again adjusted to a pH4 value or more, or by concentrating the permeate obtained in (1) using an ultrafiltration membrane having a fractional molecular weight of 10,000Da or less.

Patent Document 2 discloses a method for producing a composition having a high GMP content, wherein a milk whey adjusted to a pH5.0 or more is heat-treated at a temperature of 80°C or more to produce a whey protein aggregate, the aggregate is separated by a microfiltration membrane having a pore size of 0.5µm or less or an ultrafiltration membrane having a molecular weight fraction of 50,000Da or more, and the GMP is recovered in a membrane permeate.

Patent Document 3 discloses a method for producing a caseinomaceoid-containing composition having the steps of: providing a whey-derived feed containing CMP and at least one additional protein, with a maximum pH of 4; providing an enriched UF permeate and UF residue with respect to CMP by using an ultrafiltration membrane filter to pass a monomer CMP to ultrafilter the whey-derived feed; contacting the first composition derived from the UF permeate with a cation exchanger; collecting a fraction of the first composition that does not bind with the cation exchanger, thereby obtaining the CMP-containing composition.

Patent Document 4 discloses a process for producing a composition containing hight sialic acids and α-lactalbumin content, characterized in that whey is cooled and the pH is adjusted to 6 to 9 after Ca and/or Mg is added; the precipitate formed after heating and holding at 40 to 60°C is treated with an ultrafiltration membrane having a membrane pore diameter of 1µm or less to remove the precipitate; the pH of the obtained filtrate is adjusted to 4 or more and then treated with a membrane having a membrane pore diameter of 50,000Da or less to recover concentrate; the concentrate is adjusted to pH of 3.0 to 5.0, and then heated for 30 to 60 minutes at 40 to 50°C; the precipitate formed is recovered and the pH is adjusted to 6 to 8 to dissolve the precipitate; and the sialic acids and *α* -lactalbumin are concentrated, desalinated and powdered as required.

### RELATED ART

[Patent Document 1]
   Japanese Patent No. 2,673,828
[Patent Document 2]
   Japanese Patent No. 3,225,080
[Patent Document 3]
   Japanese Patent No. 6,396,309
[Patent Document 4]
   Japanese Patent No. 3,044,487

### SUMMARY OF INVENTION

### [Problem to be Solved by the Invention]

On the method of Example 1 in Patent Document 1, assuming that the protein concentration in 1kg of whey protein concentrate is 80% by mass and the GMP content with respect to the total protein concentration is 15% by mass, the recovery rate of GMP is 45% based on 54g of GMP obtained by lyophilization of the concentrate.

On the other hand, on the method of Example 2 in Patent Document 1, assuming that the protein concentration in 500L of Gorda cheese whey is 0.7% by mass and the GMP content relative to the total protein concentration is 15% by mass, the recovery rate of GMP is 1.1% on the basis of 5.7g GMP obtained by concentration, desalination and lyophilization. There has been a problem that the recovery rate is remarkably lowered when the starting material is cheese whey. In Patent Document 2, although the purity of the obtained GMP composition is described, the recovery rate is not described, and it is unclear.

In the method of Patent Document 3, although the recovery rate of the obtained GMP composition reaches 50%, both the UF membrane processing and the cation exchange chromatography processing are necessary, and the operation is complicated, and considerable time and labor are required to obtain the object.

Therefore, there has been a need for a method for producing a composition containing GMP, which is simple to operate and has a high yield regardless of whether the starting material is cheese whey or whey protein concentrate.

It is a first object of the present invention to provide a method for producing a composition containing GMP, which is simple to operate and has a high yield regardless of whether the starting material is cheese whey or whey protein concentrate.

The arts disclosed in Patent Documents 1 to 3 require many essential steps and complicated operations, and require considerable time and labor to obtain an object. Therefore, there has been a need for a novel method for producing a composition containing GMP, which is not known in the art and which can solve the above-mentioned problems.

It is a second object of the present invention to provide a method for producing a composition containing GMP, which is easy to operate and shortens the production time.

Further, in the techniques disclosed in Patent Documents 1 to 3, obtaining LA is not disclosed at all, and a large number of steps are required to obtain an object. Patent Documents 3 and 4, which disclose that both GMP and LA are recovered, have many essential steps, are extremely complicated to operate, and require a considerable amount of time and labor to obtain an object. Therefore, there has been a need for a novel method for producing a composition containing GMP and LA, which is not known in the art and which can solve the above-mentioned problems.

A third object of the present invention is to provide a method for producing a composition containing GMP and LA, which is easy to operate and shortens the production time.

### SOLUTION TO PROBLEM

### [A. First embodiment]

It is said that GMP have a molecular weight of about 9,000Da, which present as monomers at pH of less than 4 and polymers (molecular weight 50,000Da or more) at pH of 4 or more. On the other hand, as a result of intensive investigation by the inventors, it was found that a composition containing GMP was obtained from a permeate obtained by treatment with a membrane having a molecular weight fraction of 9,000 to 50,000Da, even though the pH was 4 or more, wherein the composition is produced by preparing an aqueous solution of a milk raw material containing GMP and having a pH of 4 to 9, and generating hydrogen carbonate ions and/or carbonate ions and metal ions in the aqueous solution.

Specifically, the present invention includes the following configurations.
[1] A method for producing a composition containing GMP, including: (A) preparing an aqueous solution containing a milk raw material having a pH of 4 to 9; (B) pretreating the aqueous solution to obtain an pretreated aqueous solution containing carbonate (hydrogen) ions and metal ions; and (C) subjecting the pretreated aqueous solution to membrane treatment using a membrane having a molecular weight fraction of 9,000Da or more and 300,000Da or less.
[2] The method for producing a composition according to [1], wherein the composition containing GMP is obtained as the permeate of the step (C).
[3] The method for producing a composition according to [1] or [2], wherein the method includes adjusting a pH of an aqueous solution containing a milk raw material having pH of less than 4 to pH of 4 or more between the steps (B) and (C).
[4] The method for producing a composition according to any one of [1] to [3], further including (D) subjecting the concentrate obtained in the step (C) to the membrane treatment again to obtain a re-permeate obtained by this treatment.
[5] The method for producing the composition according to [4], further including (E) concentrating the permeate and/or the re-permeate using a membrane having a molecular weight fraction of less than 9,000Da.
[6] The method for producing the composition according to any one of [1] to [5], wherein the fractional molecular weight of the membrane used in step (C) is 30,000Da to 100,000Da.

### [B.Second embodiment]

It is said that GMP have a molecular weight of about 9,000Da, which present as monomers at pH of less than 4 and polymers (molecular weight 50,000Da or more) at pH of 4 or more.

On the other hand, as a result of intensive investigation by the inventors, it was found that a composition containing GMP was obtained from a permeate obtained by treatment with a membrane having a molecular weight fraction of 9,000 to 50,000Da, even though the pH was 4 or more, wherein the composition is produced by preparing an aqueous solution of a milk raw material containing GMP and having a pH of 4 to 9, and generating hydrogen carbonate ions and/or carbonate ions and metal ions in the aqueous solution. Specifically, the present invention includes the following configurations.
[1] A method for producing a composition containing GMP, including:
   (A) preparing an aqueous solution containing a milk raw material and having a pH of 4 or more and pH of 9 or less,
   (B) producing hydrogen carbonate ions and/or carbonate ions in the aqueous solution,
   (C) subjecting the aqueous solution in which hydrogen carbonate ions and/or carbonate ions are produced to membrane treatment using a membrane having a molecular weight fraction of 9,000 to 50,000Da.
[2] The method for producing a composition according to [1], further including (D) obtaining a composition containing GMP as the permeate of the step (C).
[3] The method for producing a composition according to [1] or [2], which does not include the step of adjusting the pH of the aqueous solution containing the milk raw material from pH of less than 4 to pH of 4 or more prior to the step (C),
[4] The method for producing a composition according to any one of [1] to [3], further including (E) subjecting the concentrate obtained in the step (C) to the membrane treatment again to obtain a re-permeate.
[5] The method for producing a composition according to any one of [1] to [4], further including (F) concentrating the permeate and/or re-permeate using a membrane having a molecular weight fraction of less than 9,000, and
[6] The method for producing a composition according to any one of [1] to [5], wherein the membrane used in the step (C) has a molecular weight fraction of 30,000 to 50,000Da.

### [C. Third Embodiment]

As a result of intensive investigation by the inventors, it was found that a composition containing GMP and LA was obtained from a permeate of membrane processing using a membrane having a molecular weight fraction of 300,000Da or less, wherein the composition is produced by preparing an aqueous solution containing GMP and LA and having a pH of 4 to 9, and generating hydrogen carbonate ions and/or carbonate ions and metal ions in the aqueous solution. Specifically, the present invention includes the following configurations.
[1] A method of making a composition including GMP and LA, including: (A) preparing an aqueous solution containing a milk raw material and having a pH of 4 or more and pH of 9 or less, (B) producing hydrogen carbonate ions and/or carbonate ions in the aqueous solution, (C) generating metal ions, (D) subjecting the aqueous solution in which the hydrogen carbonate ion and/or carbonate ion and metal ion are produced to membrane treatment using a membrane having a molecular weight fraction of 9,000 to 300,000Da.
[2] The method for producing a composition according to [1], further including: (E) obtaining a composition containing GMP and LA as the permeate of the step (D).
[3] The method for producing a composition according to [1] or [2], which does not include the step of adjusting the pH of the aqueous solution containing the milk raw material from pH of less than 4 to pH of 4 or more prior to the step (D).
[4] The method for producing a composition according to any one of [1] to [3], further including (F) subjecting the concentrate obtained in the step (D) to the membrane treatment again to obtain a re-permeate, and
[5] The method for producing a composition according to any one of [1] to [4], further including (G) concentrating the permeate and/or the re-permeate using a membrane having a molecular weight fraction of less than 9,000.

### EFFECTS OF INVENTION

The invention according to the first embodiment provides a novel method for producing a composition containing GMP, which is not conventionally available. According to the present invention, there is provided a method for producing a composition containing GMP, which is simple to operate, but has a high yield, whether the starting material is cheese whey or whey protein concentrate.

The invention according to the second embodiment provides a novel method for producing a composition containing GMP, which is not conventionally possible to solve the above-mentioned problems. According to the present invention, it is possible to recover GMP in high yield by a simple method.

The invention according to the third embodiment provides a novel method for producing a composition containing GMP and LA, which is not conventionally possible to solve the above-mentioned problems. According to the present invention, it is possible to recover GMP and LA in high yield by a simple method. Methods capable of recovering GMP and LA at the same time in high yield and with a small number of steps have not yet been reported.

### BRIEF DESCRIPTION OF THE DRAWINGS

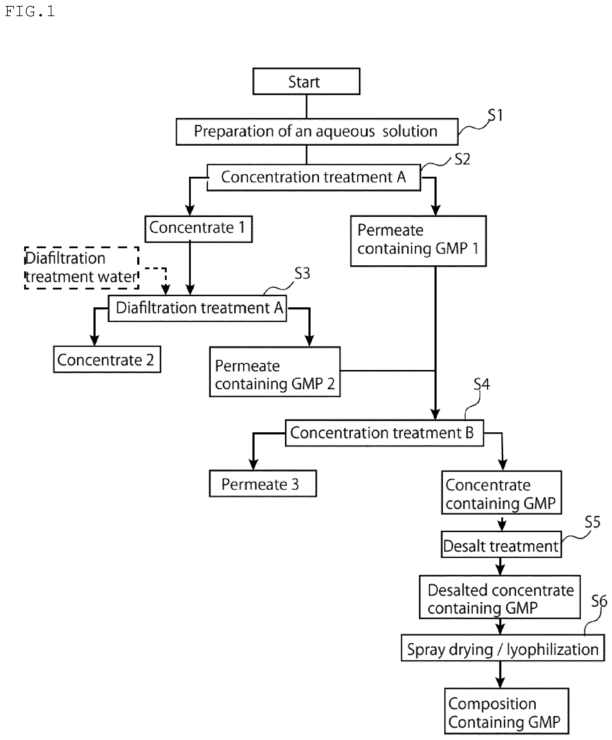

FIG. 1 is a flow chart of a method of making a composition containing GMP.

### DESCRIPTION OF EMBODIMENT

The present invention will be described with reference to the first, second, and third embodiments, but the present invention is not limited to the following embodiments.

### [A. First embodiment]

A raw material used in the method for producing the composition according to the first embodiment will be described.

### (raw material for milk)

GMP, a sialic acid, is included in whey such as a by-product of making cheese or rennet casein from milk such as cow's milk, goat's milk, and sheep's milk. Therefore, in the method for producing the composition according to the embodiment, such a milk raw material containing GMP can be used. As milk raw material, cheese whey, rennet casein whey, whey protein concentrate (WPC), whey protein isolate (WPI), demineralized cheese whey powder, non-demineralized cheese whey powder, and the like can be exemplified. Note that the same material as GMP can be used for LA.

In the method for producing a composition containing GMP according to the embodiment, the above-mentioned aqueous solution of a milk raw material is prepared. The solid content concentration at this time is preferably 0.001 % by mass or more, more preferably 0.001 % by mass or more and 35 % by mass or less, and most preferably 0.01 % by mass or more and 20 % by mass or less.

### (pH adjustment)

In the method for producing a composition containing GMP according to the embodiment, it is preferable that the pH of the aqueous solution containing the milk raw material is in the range of 4 to 9. This is because the selectivity of the permeate liquid and the concentrate liquid in the concentration processing step is enhanced by satisfying the pH condition, the condition of the carbonate (hydrogen) ion and the metal ion, and the condition of the permeate membrane, which will be described later.

When the pH of the aqueous solution containing the milk raw material is within the range of 4 to 9, it is not necessary to adjust the pH, but when the pH of the aqueous solution containing the milk raw material is less than 4 or more than 9, the pH may be adjusted to 4 to 9. However, from the viewpoint of maintaining the chemical structure or stability of GMP, it is preferable not to include such a pH adjustment step. For example, in the method for producing a composition containing GMP of the present invention, it is preferable that the step of once adjusting the pH of an aqueous solution containing a milk raw material to pH of less than 4 and again adjusting the aqueous solution to pH of 4 or more is not included. Similarly, it is preferable not to include a step of once adjusting the pH of the aqueous solution containing the milk raw material pH of above 9 and again adjusting the aqueous solution to pH of 9 or less.

When the pH becomes 4 or less after carbonate (hydrogen) ions and metal ions are produced in an aqueous solution containing a milk raw material and having a pH of 4 to 9, it is preferable to readjust the pH to 4 or more.

The pH can be adjusted by any method that can be used in the manufacture of foods and pharmaceuticals for oral ingestion. The use of one or more acids, such as hydrochloric acid, sulfuric acid, lactic acid, and the like, as reagents for adjusting the pH from above 9 to below 9, and the use of one or more bases, such as sodium hydroxide, potassium hydroxide, and the like, as reagents for adjusting the pH from below 4 to above 4, can be exemplified. However, a reagent which produces hydrogen carbonate ion and/or carbonate ion is not preferable because the amount of GMP in the obtained composition is lowered when the pH is adjusted from above 9 to pH of 9 or less by using the reagent.

As noted above, the pH may be 4 to 9, preferably pH of 4 to 9, more preferably pH of 4. 5 to 8.5, more preferably pH of 5. 0 to 8.5, more preferably pH of 5. 5 to 8.5, and most preferably pH of 6. 0 to 8.5.

### (Carbonate hydrogen ions and/or carbonate ions)

In the method for producing a composition containing GMP according to the embodiment, hydrogen carbonate ions and/or carbonate ions are produced in an aqueous solution containing a milk raw material and having a pH of 4 to 9. The hydrogen carbonate ions and/or carbonate ions may be generated simultaneously with the preparation of an aqueous solution containing the milk raw material and having a pH of 4 to 9, but are preferably generated after the preparation in order to prevent the disappearance of the hydorgen carbonate and/or carbonate ions.

In the present specification and claims, the term "carbonate (hydrogen) ion" includes carbonate ion or hydrogen carbonate ion, or both.

The hydrogen carbonate ions and/or carbonate ions may be produced by using any component or method that produces carbonate and/or hydrogen carbonate ions in aqueous solution, and may be produced by combining one or more of the compounds and/or methods exemplified below to produce carbonate ion and/or hydrogen carbonate ion.

As the method for the above, a method of adding carbon dioxide such as blowing of gaseous carbon dioxide, addition of liquid or solid carbon dioxide; a method of adding a hydrogen carbonate such as sodium hydrogen carbonate, potassium hydrogen carbonate, calcium hydrogen carbonate, or ammonium hydrogen carbonate; and a method of adding a carbonate such as sodium carbonate, potassium carbonate, ammonium carbonate, or calcium carbonate can be exemplified.

When a hydrogen carbonate or carbonate is added, it may be possible to dissolve these salts into water so as to prepare an aqueous solution in which carbonate ions and/or hydrogen carbonate ions are generated, and then add the prepared aqueous solution to an aqueous solution containing a milk raw material; or, it may be possible to directly introduce hydrogen carbonate or carbonate into an aqueous solution containing a milk raw material.

In the process for producing a composition containing GMP according to the embodiment, a solution containing a milk raw material containing GMP and having pH of 4 to 9 is prepared, and hydrogen carbonate ions and/or carbonate ions are produced in the solution in the aqueous solution. The concentration of the hydrogen carbonate ions and/or carbonate ions in the solution can be 0.001% by mass or more and 5% by mass or less. Specific ranges can be 0.001 to 5 % by mass, 0.0025 to 3 % by mass, 0.005 to 2 % by mass, 0.0075 to 2 % by mass, 0.01 to 2 % by mass, 0.025 to 2 % by mass, or 0.05 to 2 % by mass.

### (metal ion)

The light metal salt and/or transition metal salt used in the embodiment will be described. As light metal salt and/or transition metal salt, it is possible to use any one containing a monovalent to trivalent cations of light metal or transition metal, it is possible to use one or more metal ions. Examples of the light metal ion and/or the transition metal ion contained in the light metal salt and/or the transition metal salt include sodium, potassium, magnesium, calcium, manganese, iron, copper, zinc, and the like, and it is preferable to use sodium, potassium, magnesium, calcium, manganese, iron, copper, zinc, and the like which are present in foods and humans. Types of light metal salts and/or transition metal salts include hydrochlorides, sulfates, nitrates, acetates, lactates, carbonates, oxalates, phosphates, and the like.

As the metal ion source, an inorganic salt or an organic salt of a light metal and/or a transition metal, a mineral material derived from milk, or whey minerals generated during the production of WPI and/or WPC can be used. When cheese whey or rennet casein whey is used as a raw material, or when a raw material obtained by adding a raw material containing GMP to acid whey is used, a permeate containing whey mineral obtained when GMP is prepared by pretreatment with an ultrafiltration membrane or the like having a fractional molecular weight smaller than 9,000 may be used.

When light metal salts and/or transition metal salts are added, it may be possible to dissolve these salts into an aqueous solution so as to prepare an aqueous solution in which metal ions are generated, and then add the prepared aqueous solution to an aqueous solution containing a milk raw material; or, it may be possible to directly introduce light metal salts and/or carbonates to an aqueous solution containing a milk raw material so as to generate hydrogen carbonate ions and/or carbonate ions and metal ions simultaneously. When metal ions are generated in an aqueous solution containing a milk raw material, it may be possible to generate hydrogen carbonate ions and/or carbonate ions and metal ions simultaneously in an aqueous solution containing the milk raw material and adjusted to a pH of 4 to 9. When, hydrogen carbonate ions and/or carbonate ions and metal ions are not generated simultaneously, it is preferable to generate metal ions after the hydrogen carbonate ions and/or carbonate ions are generated.

Specifically, the lower limit of the metal ion in the solution may be 0.0001% by mass or more, 0.00025% by mass or more, 0.0005% by mass or more, 0.00075% by mass or more, or 0.001% by mass or more. The upper limit of the concentration of the metal ions in the solution may be 5% by mass or less, 3% by mass or less, or 2% by mass or less. Specific ranges may be from 0.0001 to 5 % by mass, from 0.00025 to 3 % by mass, from 0.0005 to 2 % by mass, from 0.00075 to 2 % by mass, or from 0.001 to 2 % by mass.

### (Separation Membrane)

In the method for producing a composition containing GMP according to the embodiment, a membrane treatment is performed on an aqueous solution containing a milk raw material and having a pH of 4 to 9 in which hydrogen carbonate ions and/or carbonate ions and light metal salts and/or transition metal salts are formed. The membrane used for membrane treatment can be any membrane having a molecular weight fraction of 9,000 to 300,000Da, preferably 10,000 to 300,000Da, more preferably 20,000 to 100,000Da, and most preferably 30,000 to 100,000Da.

As the material of the membrane, polysulfone, polyethersulfone, ethylene tetrafluoride, ceramic, hydrophilic membrane such as cellulose acetate, nitrocellulose, polyacrylonitrile, aromatic polyamide, or charged membrane can be exemplified.

### (Membrane Treatment Method)

The membrane treatment method used in the method for producing the composition containing GMP according to the embodiment may be any method commonly used in the treatment, manufacture, or the like of a food or a medicine to be ingested orally. Filtration treatment, diafiltration, or the like in a cross flow method can be exemplified. The permeate in this membrane treatment can be obtained as a composition containing GMP. Note that the temperature of the solution during the membrane treatment need not be adjusted, but it is preferable to set the temperature to 0°C or more and 15°C or less in consideration of the propagation of microorganisms.

### (An aspect of a method for producing a composition containing GMP)

### (Flow chart)

FIG. 1 is a flow chart of a method of making a composition containing GMP.

A method for producing a composition containing GMP will be described with reference to the flow chart of FIG. 1. Although the method of FIG. 1 is used as example for the description, the present invention is not limited to this method.

### (1) Pretreatment of aqueous solution containing milk raw material (Step 1)

The milk raw material is dissolved in water to prepare an aqueous solution containing GMP.

In the aqueous solution containing GMP, it is preferable to adjust the concentration of GMP by pretreatment with an ultrafiltration membrane or the like having a fractional molecular weight smaller than 9,000Da before pH adjustment of the aqueous solution containing GMP and formation of hydrogen carbonate ions and/or carbonate ions in the aqueous solution containing GMP.

In addition, it is preferable to remove fats, insolubles, and the like by using a pretreatment such as a cream separator or a clarifier. It is also possible to heat-treat an aqueous solution containing a milk raw material or GMP for the purpose of sterilization.

### (2) Concentration of an aqueous solution containing a milk raw material (Step 2)

Concentration of the aqueous solution containing GMP is performed (Concentration Process A). Thereby, permeate containing GMP and a concentrate 1 are obtained. The membrane used in the concentration process can have any molecular weight fraction of 9,000 to 300,000Da, preferably 10,000 to 300,000Da, more preferably 20,000 to 100,000Da, and most preferably 30,000 to 100,000Da.

### (3) Diafiltration of concentrate (Step 3)

One method of improving the yield of GMP is diafiltration (hereinafter also referred to as "DF") treatment of concentrate 1.

By the DF treatment in which the membrane treatment is performed while water or the like is added to the concentrate 1, the GMP remaining in the concentrate 1 can be recovered from the permeate (permeate containing GMP 2).

It is preferable to recover GMP as much as possible from the re-permeate (permeate containing GMP 2) by repeating this process.

As the water addition to the concentrated solution, filtered water, ion-exchanged water, distilled water, ultrapure water, a permeate obtained when a permeate containing GMP is concentrated by membrane treatment, a solution having adjusted pH or ionic strength, a mixture of these, or the like can be used.

### (4) Concentration and/or demineralization of GMP-containing permeate (steps 4, 5)

Since the composition containing GMP obtained as the permeate of the membrane treatment (the permeate containing GMP 1 and the permeate containing GMP 2) contains lactose and minerals in addition to the target GMP. It is possible to concentrate and desalt by using an ultrafiltration membrane having a fractional molecular weight smaller than 9,000Da. That is, it is possible to remove lactose and minerals. This makes it possible to further increase the GMP content in the composition. The fractional molecular weight of the ultrafiltration membrane for concentration and/or desalting is preferably 5,000Da or less.

### (5) Spray drying and/or lyophilization of the composition containing GMP (step 6)

In addition, compositions containing GMP with reduced moisture can be obtained by means such as spray drying and/or lyophilization.

### (Method for Determining GMP Content of Composition Containing GMP)

The GMP content in the raw material used for the treatment and the GMP content in the composition containing the GMP obtained as a result of the treatment were measured using urea-SDS electrophoresis.

### EXAMPLE A

Hereinafter, examples of the invention according to the first embodiment will be described in detail, but the present invention is not limited thereto. The description will be made with reference to FIG. 1.

### Example A1: Preparation of a Composition Containing GMP from Cheese Whey

Step 1: 40kg of cheddar cheese whey were placed on a clarifier after pasteurization at 75°C to remove insolubles. After removal of insoluble matter, the cheddar cheese whey was concentrated 4-fold using an ultrafiltration membrane with a molecular weight fraction of 5,000Da to obtain 10kg concentrate and 30kg permeate.
   sodium hydrogen carbonate was added to 10kg of this cheddar cheese whey concentrate and stirred well. Thereafter, 2.5kg of the permeate was added to prepare 12.5kg of desalted cheddar cheese whey having a sodium hydrogen carbonate concentration of 0.42 % by mass. The pH of the 0.42% by mass sodium hydrogen carbonate-desalted cheddar cheese whey was 8.4.
Step 2: The aqueous solution obtained in Step 1 was subjected to a cross-flow filtration process using a filtration membrane (polysulfone material, molecular weight fraction 50,000Da, membrane area 0.2m²). The filtration process was performed at a temperature of 10°C and a mean operating pressure of 0.2MPa. This process resulted in a concentrate of 6.25kg and a permeate of 6.25kg (composition containing GMP). The pH of the obtained permeate was 7.5.
Step 3: DF treatment was performed on 6.25kg of the concentrate obtained in Step 2. The filtration membrane used and the operating conditions are the same as described in step 2. The DF treatment was terminated when the weight of the retentate (composition containing GMP) reached 18.75kg.
Step 4: A total of 25kg (pH7.6) of the permeate and retentate obtained in steps 2 and 3 was concentrated by a cross-flow method using a filter membrane (polyethersulfone material, molecular weight fraction: 5,000Da, membrane area: 0.33 m²). The treatment conditions were a temperature of 10°C and a mean operating pressure of 0.4MPa, and 5kg (composition containing GMP) of the concentrate and 20kg of the permeate were obtained.
Step 5: Next, a DF treatment of 5kg of concentrate with GMP obtained in Step 4 was carried out. The filtration membrane used and operating conditions were the same as in step 4. The DF treatment was terminated when the permeate became 10kg.
Step 6: 5kg of the concentrate containing GMP after the DF treatment was freeze-dried to obtain 75g of GMP in powder form. The content of GMP in dry powder form was 37% by mass per solid content. The recovery rate of GMP from the raw material used was 50% by mass.

### (Example A2) : Preparation of a Composition containing GMP from A whey Protein Purification (WPI) and a whey Mineral Material

Step 1: An 8% by mass WPI aqueous solution was prepared using Provon190 (Glanbia Nutritionals) from cheese whey as WPI, and a 2% by mass whey mineral aqueous solution was prepared by using FondlacSL (Meggle AG) as whey mineral material. FondlacSL ash analyses were 5024mg of K, 1704mg of Na, 252mg of Mg and 1044mg of Ca per 100g. A sodium hydrogen carbonate aqueous solution was prepared by adding sodium hydrogen carbonate to ion-exchanged water so that the concentration of the aqueous solution was 1.68 % by mass.
   Next, 3.75kg of a 1.68 % by mass sodium hydrogen carbonate aqueous solution was added to 3.75kg of an 8 % by mass aqueous solution of WPI and stirred well. Thereafter, 7.5kg of a 2 % by mass whey mineral aqueous solution was added, and 15kg of 2 % by mass WPI-0. 42 % by mass sodium hydrogen carbonate-1 % by mass whey mineral aqueous solution was prepared. The pH of the 2 % by mass WPI-0. 42 % by mass sodium hydrogen carbonate-1 % by mass whey mineral aqueous solution was 8.2.
Step 2: The aqueous solution was subjected to a filtration treatment by a cross flow method using a filtration membrane (polyethersulfone material, molecular weight fraction 50,000Da, membrane area 0.33 m²). The condition of the filtering treatment was a temperature of 10°C and a mean operating pressure of 0.2MPa. This treatment gave 5kg of concentrate and 10kg of permeate (a composition containing GMP). The pH of the obtained permeate was 7.5.
Step 3: DF treatment was performed on 5kg of the concentrate obtained in Step 2. The filtration membrane used and the operating conditions are the same as described in step 2. The DF treatment was terminated when the weight of the retentate (composition containing GMP) reached 15kg.
Step 4: A total of 25kg (pH7. 5) of the permeate and re-permeate obtained in Steps 2 and 3 was concentrated by a cross-flow method using a filter membrane (a polyethersulfone material, a molecular weight fraction of 5,000Da, and a membrane area of 0.33 m²). The treatment conditions were 10°C in temperature and 0.4MPa in mean operating pressure. This treatment gave 5kg of concentrate (a composition containing GMP), and 20kg of permeate.
Step 5: Subsequently, the DF treatment of the concentrate containing GMP obtained in step 4 was performed. The filtration membrane used and operating conditions were the same as in step 4. The DF treatment was terminated when the permeate became 15kg.
Step 6: 5kg of the concentrate containing GMP after the DF treatment was freeze-dried to obtain 86g of a composition containing GMP in powder form. The content of GMP in dry powder form was 36% by mass per solid content. The recovery rate of GMP from the raw material used was 50% by mass.

### (Example A3) : Preparation of a Composition Containing GMP from a whey Protein Purification (WPI) and Copper Sulfate

Step 1: An 8% by mass WPI aqueous solution was prepared using Provon190 (Glanbia Nutritionals) from cheese whey as WPI. Next, a sodium hydrogen carbonate aqueous solution was prepared by adding sodium hydrogen carbonate to ion-exchanged water so that the concentration of the aqueous solution was 3.36 % by mass. Further, an aqueous solution of copper(II) sulfate pentahydrate was prepared by adding copper(II) sulfate pentahydrate (Kanto Chemical Company) to ion exchanged water so that the concentration of the aqueous solution was 0.12 % by mass. To 3.75kg of an 8 % by mass WPI aqueous solution, 3.75kg of a 3.36 % by mass sodium hydrogen carbonate aqueous solution was added and stirred well. Thereafter, 7.5kg of a copper sulfate aqueous solution was added thereto, and 15kg of aqueous solution of 2 % by mass WPI-0. 84 % by mass sodium hydrogen carbonate-0.06 % by mass copper sulfate was prepared. The PH of the aqueous solution of 2 % by mass WPI-0. 84 % by mass sodium hydrogen carbonate-0.06 % by mass copper sulfate was 8.
Step 2: The aqueous solution was subjected to a filtration treatment by a cross-flow method using a filtration membrane (polysulfone material, molecular weight fraction: 100,000Da, membrane area: 0.2m²). The condition of the filtering treatment was a temperature of 10°C and a mean operating pressure of 0.2MPa. This treatment gave 5kg of concentrate and 10kg of permeate, a composition containing GMP.
Step 3: DF treatment was performed on 5kg of the concentrate obtained in Step 2. The filtration membrane used and the operating conditions are the same as described in step 2. The DF treatment was terminated when the weight of the retentate (composition containing GMP) reached 40kg.
Step 4: 50kg (pH7.3) of the permeate and re-permeate obtained in steps 2 and 3 were concentrated by a cross-flow method using a filter membrane (polyethersulfone material, molecular weight fraction of 5,000Da, membrane area of 0.33 m²). The treatment conditions were 10°C in temperature and 0. 4MPa in mean operating pressure. This treatment gave 5kg of concentrate (a composition containing GMP), and 45kg of permeate.
Step 5: Next, a DF treatment of 5kg of concentrate with GMP obtained in Step 4 was carried out. The filtration membrane used and operating conditions were the same as in step 4. The DF treatment was terminated when the weight of the permeate reached 10kg.
Step 6: 5kg of the concentrate containing GMP after the DF treatment was freeze-dried to obtain 42g of a composition containing GMP in powder form. The content of GMP in dry powder form was 40% by mass per solid content. The recovery rate of GMP from the raw material used was 31% by mass.

### (Example A4) : Preparation of a Composition Containing GMP from whey Protein Purification (WPI) and Ferrous Chloride

Step 1: An 8% by mass WPI aqueous solution was prepared using Provon190 (Glanbia Nutritionals) from cheese whey as WPI. Next, a sodium hydrogen carbonate aqueous solution was prepared by adding sodium hydrogen carbonate to ion-exchanged water so that the concentration of the aqueous solution was 3.36 % by mass. Further, an aqueous solution of iron(III) chloride hexahydrate was prepared by adding iron(III) chloride hexahydrate (Pure Chemical Company) to ion exchanged water so that the concentration of the aqueous solution was 0.14 % by mass. To 3.75kg of an 8 % by mass WPI aqueous solution, 3.75kg of a 3.36 % by mass sodium hydrogen carbonate aqueous solution was added and stirred well. Thereafter, 7.5kg of an aqueous solution of iron chloride was added thereto to prepare 15kg of an aqueous solution of 2% by mass WPI-0. 84% by mass sodium hydrogen carbonate-0.07% by mass iron chloride. The pH of the aqueous solution of 2 % by mass WPI-0. 84 % by mass sodium hydrogen carbonate-0.07 % by mass ferric chloride was 7.8.
Step 2: The aqueous solution was subjected to a filtration treatment by a cross-flow method using a filtration membrane (polysulfone material, molecular weight fraction: 100,000Da, membrane area: 0.2m²). The condition of the filtering treatment was a temperature of 10°C and a mean operating pressure of 0. 2MPa. This treatment gave 5kg of concentrate and 10kg of permeate (a composition containing GMP).
Step 3: DF treatment was performed on 5kg of the concentrate obtained in Step 2. The filtration membrane used and the operating conditions are the same as described in step 2. The DF treatment was terminated when the weight of the retentate (composition containing GMP) reached 30kg.
Step 4: 40kg (pH7.3) of the permeate and re-permeate obtained in steps 2 and 3 were concentrated by a cross-flow method using a filter membrane (polyethersulfone material, molecular weight fraction of 5,000Da, membrane area of 0.33 m²). The treatment conditions were 10°C in temperature and 0. 4MPa in mean operating pressure. This treatment gave 5kg of concentrate (a composition containing GMP), and 35kg of permeate.
Step 5: Next, a DF treatment of 5kg of concentrate with GMP obtained in Step 4 was carried out. The filtration membrane used and operating conditions were the same as in step 4. The DF treatment was terminated when the permeate became 15kg.
Step 6: 5kg of the concentrate containing GMP after the DF treatment was freeze-dried to obtain 71g of a composition containing GMP in powder form. The content of GMP in dry powder form was 28% by mass per solid content. The recovery rate of GMP from the raw material used was 37% by mass.

### [B.Second embodiment]

The second embodiment of the present invention will be explained focusing on the difference from the first embodiment described above.

### (pH adjustment)

In the method for producing a composition containing GMP according to the embodiment, the step of adjusting the pH of the aqueous solution containing the milk raw material from outside range of 4 to 9 to the range of 4 to 9 can be included, but from the viewpoint of maintaining the chemical structure or stability of GMP, it is preferable not to include such a step. For example, in the method for producing a composition containing GMP of the present invention, it is preferable that the step of once adjusting the pH of an aqueous solution containing a milk raw material to less than 4 and again adjusting the aqueous solution to 4 or more is not included. Similarly, it is preferable not to include a step of once adjusting the pH of the aqueous solution containing the milk raw material above 9 and again adjusting the aqueous solution to 9 or less.

In the method for producing a composition containing GMP according to the embodiment, when the pH of the aqueous solution is within the range of 4 to 9, it is not necessary to adjust the pH within the range of 4 to 9. However, if the pH is less than 4 or greater than 9, adjust the pH to 4 to 9. The pH can be adjusted by any method that can be used in the manufacture of foods and pharmaceuticals for oral ingestion. The use of one or more of hydrochloric acid, sulfuric acid, lactic acid, and the like can be exemplified as a reagent for adjusting the pH from above 9 to below 9, and the use of one or more of sodium hydroxide, potassium hydroxide, and the like can be exemplified as a reagent for adjusting the pH from below 4 to above 4.

However, a reagent which produces hydrogen carbonate ion and/or carbonate ion is not preferable because the amount of GMP in the obtained composition is lowered when the pH is adjusted by using the reagent.

As noted above, the pH may be pH 4 to 9, preferably pH of 4 to 9, more preferably pH of 4.5 to 8.5, more preferably pH of 5 to 8, more preferably pH of 5.5 to 8, and most preferably pH of 6 to 8.

In a process for producing a GMP-containing composition according to an embodiment, a solution of pH of 4 to 9 containing a solid content is prepared, and hydrogen carbonate ions and/or carbonate ions are produced in the solution in the aqueous solution. At this time, the concentration of the hydrogen carbonate ion and/or carbonate ion in the solution may be 0.001% by weight or more and 35% by weight or less.

The concentration of the hydrogen carbonate ions and/or carbonate ions in the solution can specifically be 0.001% by weight or more and 5% by weight or less. Specific ranges may be 0.001 to 5% by weight, 0.0025 to 3% by weight, 0.005 to 2% by weight, 0.0075 to 2% by weight, 0.01 to 2% by weight, 0.025 to 2% by weight, or 0.05 to 2% by weight.

### (Separation Membrane)

In the method for producing a composition containing GMP according to the embodiment, a membrane treatment is performed on a solution containing a milk raw material and having a pH of 4 to 9 in which hydrogen carbonate ions and/or carbonate ions are generated. Membranes used in membrane processing can have any molecular weight fractionation 9,000 to 50,000Da, preferably 10,000 to 50,000Da, more preferably 20,000 to 50,000Da, and most preferably 30,000 to 50,000Da.

The material of the membrane can be exemplified by polysulfone, polyethersulfone, ethylene tetrafluoride, ceramic, and the like, and in order to increase the yield, it is preferable to use a hydrophilic membrane such as cellulose acetate, nitrocellulose, polyacrylonitrile, or aromatic polyamide, or a charged membrane.

(An aspect of a method for producing a composition containing GMP)

### (1) Diafiltration (DF) processing method

In order to obtain GMP, when a permeate containing GMP is obtained from a raw material containing GMP in membrane processing, it is preferable to obtain a re-permeate as much as possible in order to improve yield. More specifically, it is more preferable to add water to the concentrate produced in step (C), ultrafiltrate it again, and repeat this to obtain a re-permeate. As the addition of water to the concentrated solution, filtered water, ion-exchanged water, distilled water, ultrapure water, a permeate obtained when a permeate containing GMP is concentrated by membrane treatment, a solution having adjusted pH or ionic strength, or a mixture thereof can be used.

### (2) Pretreatment of an aqueous solution containing a milk raw material

In the aqueous solution containing the milk raw material, it is preferable to adjust the concentration of GMP by pretreatment with an ultrafiltration membrane or the like having a fractional molecular weight smaller than 9,000Da before pH adjustment of the aqueous solution and formation of hydrogen carbonate ions and/or carbonate ions in the aqueous solution.

In addition, it is preferable to remove fat content, insoluble matter, and the like in the raw material by using a pretreatment such as a cream separator or a clarifier. It is also possible to heat treat these raw materials for the purpose of sterilization.

### (3) Concentration and/or desalting after step (C)

Since the composition containing GMP obtained as the permeate of the membrane treatment contains lactose and minerals in addition to the intended GMP, it is possible to concentrate and desalt using an ultrafiltration membrane having a fractional molecular weight smaller than 9,000Da. That is, it is possible to remove lactose and minerals. This makes it possible to further increase the GMP content. The fractional molecular weight of the ultrafiltration membrane for concentration and/or desalting is preferably 5,000Da or less.

### (4) Spray drying and/or freeze drying of compositions containing GMP

In addition, compositions containing GMP with reduced moisture can be obtained by means such as spray drying and/or lyophilization.

### (5) Method for measuring GMP Contents of Compositions containing GMP

The GMP content in the raw material used for the treatment and the GMP content in the composition containing the GMP obtained as a result of the treatment were measured using urea-SDS electrophoresis.

The second embodiment preferably does not include metal ions.

### EXAMPLE B

Hereinafter, examples of the invention according to the second embodiment will be described in detail, but the present invention is not limited thereto.

### (Example B1): Preparation of a Composition Containing GMP from Cheese Whey

44L of cheddar cheese whey was pasteurized at 75°C and then clarified to remove insoluble matter. After removal of insolubles, the cheddar cheese whey was concentrated twice using an ultrafiltration membrane with a molecular weight fraction of 5,000Da to obtain 20L of concentrate and 20L of permeate. To 20L of this cheddar cheese whey concentrate, 20L of a potassium hydrogen carbonate aqueous solution was added to prepare 40L of cheddar cheese whey having a concentration of 0.2 % by mass of potassium hydrogen carbonate.

The aqueous solution was subjected to a filtration treatment by a cross-flow method using a filtration membrane (polysulfone material, molecular weight fraction of 30,000Da, membrane area of 0.2m²). The condition of the filtering treatment was a temperature of 10°C and a mean operating pressure of 0.2MPa. This treatment gave 5kg of concentrate and 35kg of permeate (a composition containing GMP) . The pH of the resulting permeate was 7.3.

The 35kg of this permeate was concentrated by a cross flow method using a filtration membrane (polyethersulfone material, molecular weight fraction: 5,000Da, membrane area: 0.33 m²). The treatment conditions were 10°C in temperature and 0. 4MPa in mean operating pressure. This treatment gave 5kg of concentrate (a composition containing GMP), and 30kg of permeate.

Subsequently, 5kg of the concentrate containing GMP obtained in the preceding paragraph was subjected to DF treatment. The filtration membranes used and the operating conditions are the same as in the previous paragraph. The DF treatment was terminated when the weight of permeate reached 10kg.

After the DF treatment, 5kg of the concentrate containing GMP was freeze-dried to obtain 32g of powdery GMP.

The content of GMP in dry powder form was 39% by mass per solid content. The recovery rate of GMP from the raw material used was 19% by mass.

### (Example B2) : Preparation of a composition containing GMP from Whey Protein Purifier (WPI)

A 4%WPI aqueous solution was prepared by using Provon 190(Glanbia Nutritionals) from cheese whey as WPI. An sodium hydrogen carbonate aqueous solution was prepared by adding sodium hydrogen carbonate to ion-exchanged water so that the concentration of the aqueous solution was 0.33 % by mass. 7.5kg of a 0.33 % by mass sodium hydrogencarbonate aqueous solution was added to 7.5kg of a 4% WPI aqueous solution and stirred well to prepare 15kg of aqueous solution of 2% WPI-0.16 % by mass sodium hydrogen carbonate. The pH of the aqueous solution of 2% WPI-0. 16 % by mass sodium hydrogen carbonate was 7.2.

This aqueous solution was subjected to filtration treatment by a cross flow method using a filtration membrane (polyethersulfone material, molecular weight fraction 50,000Da, membrane area 0.33 m²). The condition of the filtering treatment was a temperature of 10°C and a mean operating pressure of 0.2MPa. This treatment gave 5kg of concentrate and 10kg of permeate (a composition containing GMP) . The pH of the resulting permeate was 7.3.

10kg of this permeate was concentrated by a cross flow method using a filtration membrane (polyethersulfone material, molecular weight fraction of 5,000Da, membrane area of 0.33 m²). The treatment conditions were 10°C in temperature and 0. 4MPa in mean operating pressure. This treatment gave 2kg of concentrate (a composition containing GMP), and 8kg of permeate.

Subsequently, DF treatment of the concentrate containing GMP obtained in the preceding paragraph was performed. The filtration membranes used and the operating conditions are the same as in the previous paragraph. The DF treatment was terminated when the permeate became 18kg. The obtained concentrate containing GMP was 2kg.

After the DF treatment, 2kg of the concentrate containing GMP was freeze-dried to obtain 44g of a composition containing GMP in powder form.

The content of GMP in dry powder form was 30% by mass per solid content. The recovery rate of GMP from the raw material used was 22% by mass.

### (Example B3) :Preparation of Compositions Containing GMP from Whey Protein Concentrate (WPC)

A 6%WPC aqueous solution was prepared by using WPC80 (Saxon Milch Company)from cheese whey as WPC. Next, a sodium hydrogen carbonate aqueous solution was prepared by adding sodium hydrogen carbonate to ion-exchanged water so that the concentration of the aqueous solution was 0.33 % by mass. To 7.5kg of a 6% aqueous solution of WPC80, 7.5kg of a 0.33% by mass sodium hydrogen carbonate aqueous solution was added and stirred well to prepare 15kg of aqueous solution of 3% WPC-0. 16% by mass of sodium hydrogen carbonate. The pH of the 6% WPC aqueous solution was 6.7, and pH of the aqueous solution of 3% WPC-0.16% by mass sodium carbonate was 7.2.

This aqueous solution was subjected to filtration treatment by a cross flow method using a filtration membrane (polyethersulfone material, molecular weight fraction 30,000Da, membrane area 0.33 m²). The condition of the filtering treatment was a temperature of 10°C and a mean operating pressure of 0.2MPa. This treatment gave 5kg of concentrate and 10kg of permeate (a composition containing GMP) . The pH of the resulting permeate was 7.3.

DF treatment was performed on 5kg of the concentrate obtained in the preceding paragraph. The filtration membranes used and the operating conditions are the same as described in the preceding paragraph. The DF treatment was terminated when the weight of the re-permeate (the composition containing GMP) reached 15kg.

25kg of the permeate and the re-permeate obtained in the previous paragraph and the previous paragraph were concentrated by a cross flow method using a filtration membrane (polyethersulfone material, molecular weight fractionation of 5,000Da, membrane area of 0.33 m²). The treatment conditions were 10°C in temperature and 0.4MPa in mean operating pressure. This treatment gave 2kg of concentrate (a composition containing GMP), and 23kg of permeate.

Subsequently, DF treatment of the concentrate containing GMP obtained in the preceding paragraph was performed. The filtration membranes used and the operating conditions are the same as in the previous paragraph. The DF treatment was terminated when the weight of the permeate reached 6kg. The obtained concentrate containing GMP was 2kg.

After the DF treatment, 2kg of the concentrate containing GMP was freeze-dried to obtain 64g of a composition containing GMP in powder form.

The content of GMP in dry powder form was 30% by mass per solid content. The recovery rate of GMP from the raw material used was 25% by mass.

### (Example B4) Preparation of Compositions Containing GMP from Rennet Casein Whey

105L of rennet casein whey adjusted to pH7.2 using sodium hydroxide was kept at 75°C for 15 seconds to sterilize, and then applied to a clarifier to remove insoluble matter. After the insoluble matter was removed, the solution was concentrated twice using an ultrafiltration membrane having a molecular weight fractionation of 5,000Da to obtain a concentrate of 50L and a permeate of 50L. The pH of the rennet casein whey concentrate after the concentration treatment was 7.3 and the Brix was 6.2%. To 50L of this rennet casein whey concentrate, 25L of a potassium hydrogen carbonate aqueous solution was added to prepare 75L of rennet casein whey of Brix6. 7% and 0.1% by mass of potassium hydrogen carbonate.

This aqueous solution was subjected to a filtration treatment by a cross-flow method using a filtration membrane (polysulfone material, molecular weight fraction 50,000Da, membrane area 0.2m²). The condition of the filtering treatment was a temperature of 10°C and a mean operating pressure of 0.2MPa. This treatment gave 5kg of concentrate and 70kg of permeate (a composition containing GMP) . The pH of the resulting permeate was 7.3.

DF treatment was performed on 5kg of the concentrate obtained in the preceding paragraph. The filtration membranes used and the operating conditions are the same as described in the preceding paragraph. The DF treatment was terminated when the weight of the retentate (composition containing GMP) reached 30kg.

100kg of the liquid (composition containing GMP) in a combination of 70kg of the permeate liquid obtained in the previous paragraph and 30kg of the re-permeate liquid obtained in the previous paragraph was subjected to concentration treatment in a cross-flow manner using a filter membrane (polyether sulfone material, fraction molecular weight 5000 Da, membrane area 0.33 m²). The treatment conditions were 10°C in temperature and 0. 4MPa in mean operating pressure. This treatment gave 5kg of concentrate (composition containing GMP) and 95kg of permeate.

Subsequently, 5kg of the concentrate containing GMP obtained in the preceding paragraph was subjected to DF treatment. The filtration membranes used and the operating conditions are the same as in the previous paragraph. The DF treatment was terminated when the permeate became 15kg.

5kg of the concentrate containing GMP after the DF treatment was subjected to a lyophilization treatment to obtain 56g of a composition containing GMP in powder form.

The content of GMP in dry powder form was 48% by mass per solid content. The recovery rate of GMP from the raw material used was 20% by mass.

### [C. Third Embodiment]

The third embodiment of the present invention will be explained focusing on the difference from the first embodiment described above.

### (pH adjustment)

In the method for producing a composition containing GMP and LA according to the embodiment, the step of adjusting the pH of the aqueous solution containing the milk raw material from an outside range of 4 to 9 to the range of 4 to 9 can be included, but from the viewpoint of maintaining the chemical structure or stability of GMP, it is preferable not to include such a step. For example, in the method for producing a composition containing GMP of the present invention, it is preferable that the step of once adjusting the pH of an aqueous solution containing a milk raw material to less than 4 and again adjusting the aqueous solution to 4 or more is not included. Similarly, it is preferable not to include a step of once adjusting the pH of the aqueous solution containing the milk raw material above 9 and again adjusting the aqueous solution to 9 or less.

In the method for producing a composition containing GMP and LA according to the embodiment, when the pH of the aqueous solution is within the range of 4 to 9, it is not necessary to adjust the pH within the range of 4 to 9. However, if the pH is less than 4 or greater than 9, it is preferable to adjust the pH to 4 to 9. The pH can be adjusted by any method that can be used in the manufacture of foods and pharmaceuticals for oral ingestion. The use of one or more of hydrochloric acid, sulfuric acid, lactic acid, and the like as a reagent for adjusting the pH from above 9 to 9 or less can be exemplified. The use of one or more of sodium hydroxide, potassium hydroxide, and the like as a reagent for adjusting the pH from less than 4 to 4 or more can be exemplified. However, a reagent which produces hydrogen carbonate ion and/or carbonate ion is not preferable because the amount of GMP in the obtained composition is lowered when the pH is adjusted by using the reagent.

As noted above, the pH may be 4 to 9, preferably pH of 4 to 9, more preferably pH of 4. 5 to 8.5, more preferably pH of 5 to 8, more preferably pH of 5. 5 to 8, and most preferably pH of 6 to 8.

### (Carbonate hydrogen ions and/or carbonate ions)

In the process for producing the compositions containing GMP and LA according to the embodiment, a solution of pH of 4 to 9 containing solid content is prepared, and hydrogen carbonate ions and/or carbonate ions are produced in the solution in the aqueous solution. At this time, the concentration of the hydrogen carbonate ion and/or carbonate ion in the solution may be 0.001% by weight or more and 35% by weight or less.

Concentration of hydrogen carbonate and/or carbonate ions in solution is specifically 0.001 % by weight or more, 5% by weight or less. Specific range can be 0.001 to 5 % by weight, 0.0025 to 3% by weight, 0.005 to 2% by weight, 0.0075 to 2% by weight, 0.01 to 2% by weight, 0.025 to 2% by weight, or 0.05 to 2% by weight.

### (Separation Membrane)

In the method for producing a composition containing GMP and LA according to the embodiment, a membrane treatment is performed on a solution containing a milk raw material and having a pH of 4 to 9 in which hydrogen carbonate ions and/or carbonate ions are generated. The membrane used for membrane treatment may be any membrane having a molecular weight fraction of 9,000 to 300,000Da or less, preferably 20,000 to 200,000Da, more preferably 30,000 to 100,000Da, and most preferably 30,000 to 50,000Da. The upper limit may also be less than 50,000Da, for example, 45,000Da, or 40,000Da, with specific ranges being 9,000 to 45,000Da, 10,000 to 40,000Da, or 20,000 to 40,000Da membranes.

The material of the membrane can be exemplified by polysulfone, polyethersulfone, ethylene tetrafluoride, ceramic, and the like, and in order to increase the yield, it is preferable to use a hydrophilic membrane such as cellulose acetate, nitrocellulose, polyacrylonitrile, or aromatic polyamide, or a charged membrane.

### (Membrane Treatment Method)

The membrane treatment method used in the method for producing the composition containing GMP and LA according to the embodiment may be any method commonly used in the treatment, manufacture, or the like of a food or a pharmaceutical for oral ingestion, and can be exemplified by filtration treatment, diafiltration, or the like in a cross flow method. The permeate in this membrane treatment can be obtained as a composition containing GMP and LA.

Note that the temperature of the solution during the membrane treatment need not to be adjusted, but it is preferable to set the temperature to 0°C or more and 15°C or less in consideration of the propagation of microorganisms.

### (One Aspect of the Producing Method)

### (1) Diafiltration (DF) processing method

In order to obtain GMP and LA, when a permeate containing GMP and LA is obtained from a raw material containing GMP and LA in membrane processing, it is preferable to obtain a re-permeate as much as possible in order to improve the yield. More specifically, it is more preferable to add water to the concentrate produced in step (D) and ultrafiltrate it again, or repeat this to obtain a re-permeate. As the addition of water to the concentrated solution, filtered water, ion-exchanged water, distilled water, ultrapure water, a permeate obtained when a permeate containing GMP and LA is concentrated by membrane treatment, a solution having adjusted pH or ionic strength, or a mixture thereof can be used.

### (2) Pretreatment of an aqueous solution containing a milk raw material

In the aqueous solution containing the milk raw material, it is preferable to adjust the concentrations of GMP and LA by pretreatment with an ultrafiltration membrane or the like having a fractional molecular weight smaller than 9,000Da before pH adjustment of the aqueous solution and formation of hydrogen carbonate ions and/or carbonate ions in the aqueous solution.

In addition, it is preferable to remove fat content, insoluble matter, and the like in the raw material by using a pretreatment such as a cream separator or a clarifier. It is also possible to heat treat these raw materials for the purpose of sterilization.

### (3) Concentration and/or desalting after step (D)

Since the composition containing GMP and LA obtained as the permeate of the membrane treatment contains lactose and minerals in addition to the intended GMP and LA, it is possible to concentrate and desalt using an ultrafiltration membrane having a fractional molecular weight smaller than 9,000Da. That is, it is possible to remove lactose and minerals. This makes it possible to further increase the GMP and LA content. The fractional molecular weight of the ultrafiltration membrane for concentration and/or desalting is preferably 5,000Da or less. Although temperature adjustment of the solution during ultrafiltration membrane treatment is unnecessary, it is preferable to set the temperature to 0°C or more and 15°C or less in consideration of propagation of microorganisms.

### (4) Spray drying and/or freeze drying of compositions containing GMP

In addition, compositions containing GMP and LA can be obtained with reduced moisture by means such as spray drying and/or lyophilization.

### (5) GMP Content Measurement Method for Compositions Containing GMP and LA

The GMP and LA contents contained in the raw materials used for the treatment and the GMP and LA contents contained in the composition containing GMP and LA obtained as a result of the treatment were measured using urea-SDS electrophoresis.

### EXAMPLE C

Hereinafter, examples of the invention according to the third embodiment will be described in detail, but the present invention is not limited thereto.

### (Example C1) : Preparation of a Composition Containing GMP and LA from Cheese Whey

54L of Gouda cheese whey was pasteurized at 75°C and then applied to a clarifier to remove insoluble matter. sodium concentration contained in the gouda cheese whey except for the insoluble matter was 0.04 % by mass, potassium concentration was 0.16 % by mass, calcium concentration was 0.03 % by mass, magnesium concentration was 0.006 % by mass. After the insoluble matter was removed, the solution was concentrated twice by using an ultrafiltration membrane having a molecular weight fractionation of 5,000Da to obtain 25L of a concentrated solution and 25L of a permeate. The pH of the Garda cheese whey concentrate after the concentration treatment was 6.3. To 25L of the gouda cheese whey concentrate, an aqueous solution of potassium hydrogen carbonate was added. After the addition of the aqueous solution of potassium hydrogen carbonate, in order to use light metal ions and/or transition metal ions inherent in gouda cheese whey, 50L of gouda cheese whey having a Brix6.3% and a concentration of potassium hydrogen carbonate of 0.1% by mass was prepared by adding a permeate obtained by concentrating gouda cheese whey twice to a concentrate of gouda cheese whey to which an aqueous solution of potassium hydrogen carbonate.

This aqueous solution was subjected to a filtration treatment by a cross-flow method using a filtration membrane (polysulfone material, molecular weight fraction 50,000Da, membrane area 0.2m²). The condition of the filtering treatment was a temperature of 10°C and a mean operating pressure of 0. 2MPa. This treatment gave 5kg of concentrate and 45kg of permeate (a composition containing GMP and LA). The pH of the resulting permeate was 7.3.

45kg of this permeate was concentrated by a cross flow method using a filtration membrane (polyethersulfone material, molecular weight fraction of 5,000Da, membrane area of 0.33 m²). The treatment conditions were 10°C in temperature and 0.4MPa in mean operating pressure. This treatment gave 5kg of concentrate (a composition containing GMP and LA), and 40kg of permeate.

Subsequently, 5kg of the concentrate containing GMP and LA obtained in the preceding paragraph was subjected to DF treatment. The filtration membranes used and the operating conditions are the same as in the previous paragraph. The DF treatment was terminated when the weight of the permeate reached 30kg.

After the DF treatment, 5kg of the concentrate containing GMP and LA was freeze-dried to obtain 52g of a composition containing GMP and LA in powder form.

The content of GMP in dry powder form was 33% by mass per solid content, and the content of LA was 61% by mass. The content of β-lactoglobulin was 6% by mass. The recovery of GMP from the raw material used was 21%.

### (Example C2) : Preparation of a Composition Containing GMP AND LA from a whey Protein Purification (WPI)

A 4%WPI aqueous solution was prepared by using Provon190 (Glanbia Nutritionals) from cheese whey as WPI. A sodium hydrogen carbonate aqueous solution was prepared by adding sodium hydrogen carbonate to ion-exchanged water so that the concentration of the concentration aqueous solution was 0.3 % by mass. Copper sulfate aqueous solution of 0.1 % by mass was prepared using copper sulfate 5hydrate as a light metal/transition metal salt. Ion exchange water was used for the preparation of the aqueous solution of sodium hydrogen carbonate and the aqueous solution of copper sulfate. 15kg of a 0.1 % by mass copper sulfate aqueous solution was added to 15kg of a 4% WPI-0. 3 % by mass sodium hydrogen carbonate and stirred well to prepare 30kg of aqueous solution of 2% WPI-0.15 % by mass sodium hydrogen carbonate-0.05 % by mass copper sulfate . The pH of the 4% WPI aqueous solution was 6.2, and the pH of the 4% WPI-0.3% sodium hydrogen carbonate aqueous solution was 7.2. The pH of the aqueous solution of 2% WPI-0.15% by mass sodium hydrogen carbonate-0.05% by mass copper sulfate was 6.8.

The aqueous solution was subjected to a filtration treatment by a cross flow method using a filtration membrane (polyethersulfone material, molecular weight fractionation of 300,000Da, membrane area of 0.33m²). The condition of the filtering treatment was a temperature of 10°C and a mean operating pressure of 0.2MPa. This treatment gave 10kg of concentrate and 20kg of permeate (a composition containing GMP and LA). The pH of the resulting permeate was 7.3.

20kg of this permeate was concentrated by a cross flow method using a filtration membrane (polyethersulfone material, molecular weight fraction 5,000Da, membrane area 0.33 m²). The treatment conditions were 10°C in temperature and 0.4MPa in mean operating pressure. This treatment gave 2kg of concentrate (a composition containing GMP and LA), and 18kg of permeate.

Subsequently, DF treatment of the concentrate containing GMP and LA obtained in the preceding paragraph was performed. The filtration membranes used and the operating conditions are the same as in the previous paragraph. The DF treatment was terminated when the weight of the permeate reached 18kg. The obtained concentrate containing GMP and LA was 2kg.

After the DF treatment, 2kg of a concentrate containing GMP and LA was freeze-dried to obtain 106g of a composition containing GMP and LA in powder form.

The content of GMP in dry powder form was 27% by mass per solid content, and the content of LA was 57% by mass. The content of β-lactoglobulin was 7% by mass. The recovery of GMP from the raw material used was 24%.

### Example C3 Preparation of a Composition Containing GMP and LA from Whey Protein Concentrate (WPC)

A 6% aqueous solution of WPC was prepared using WPC80 (Saxen Milch AG) as WPC. A sodium hydrogen carbonate aqueous solution was prepared by adding sodium hydrogen carbonate to ion-exchanged water so that the concentration of the aqueous solution was 0.33 % by mass. To 7.5kg of a 6% WPC80 aqueous solution, 7.5kg of a 0.33% by mass sodium hydrogen carbonate aqueous solution was added and stirred well to prepare 15kg of aqueous solution of 3% WPC-0. 16% by mass sodium hydrogen carbonate. The pH of the 6% WPC aqueous solution was 6.7, and the pH of the aqueous solution of 3% WPC-0.16% by mass sodium carbonate was 7.2.

This aqueous solution was subjected to filtration treatment by a cross flow method using a filtration membrane (polyethersulfone material, molecular weight fraction 30,000Da, membrane area 0.33m²). The condition of the filtering treatment was a temperature of 10°C and a mean operating pressure of 0.2MPa. This treatment gave 5kg of concentrate and 10kg of permeate (a composition containing GMP and LA). The pH of the resulting permeate was 7.3.

DF treatment was performed on 5kg of the concentrate obtained in the preceding paragraph. The filtration membranes used and the operating conditions are the same as described in the preceding paragraph. The DF treatment was terminated when the weight of the retentate (composition containing GMP and LA) reached 15kg.

25kg of the permeate and re-permeate (composition containing GMP and LA) obtained in the preceding and preceding paragraphs were concentrated by a cross flow method using a filtration membrane (polyethersulfone material, molecular weight fraction of 5,000Da, membrane area of 0.33m²). The treatment conditions were 10°C in temperature and 0.4MPa in mean operating pressure. This treatment gave 2kg of concentrate (a composition containing GMP and LA), and 23kg of permeate.

Subsequently, DF treatment of the concentrate containing GMP and LA obtained in the preceding paragraph was performed. The filtration membranes used and the operating conditions are the same as in the previous paragraph. The DF treatment was terminated when the weight of the permeate reached 6kg. The obtained concentrate containing GMP and LA was 2kg.

After the DF treatment, 2kg of the concentrate containing GMP and LA was freeze-dried to obtain 66g of a composition containing GMP and LA in powder form.

The content of GMP in dry powder form was 30% by mass per solid content, and the content of LA was 61% by mass. The content of β-lactoglobulin was 9% by mass. The recovery of GMP from the raw material used was 27%.

### (Example C4) : Preparation of a Composition Containing GMP and LA from Rennett Casein Whey

105L of rennet casein whey adjusted to pH7.2 using sodium hydroxide was kept at 75°C for 15 seconds after heating, sterilized, and then applied to a clarifier to remove insoluble matter. After the insoluble matter was removed, the solution was concentrated twice using an ultrafiltration membrane having a molecular weight fractionation of 5,000Da to obtain a concentrate of 50L and a permeate of 50L. The pH of the rennet casein whey concentrate after the concentration treatment was 7.3. To 50L of this rennet casein whey concentrate, 25L of a potassium hydrogen carbonate aqueous solution was added to prepare 75L of rennet casein whey of Brix6. 7% and 0.1% by mass of potassium hydrogen carbonate.

This aqueous solution was subjected to a filtration treatment by a cross-flow method using a filtration membrane (polysulfone material, molecular weight fraction 50,000Da, membrane area 0.2m²). The condition of the filtering treatment was a temperature of 10°C and a mean operating pressure of 0. 2MPa. This treatment gave 5kg of concentrate and 70kg of permeate (a composition containing GMP and LA). The pH of the resulting permeate was 7.3.

DF treatment was performed on 5kg of the concentrate obtained in the preceding paragraph. The filtration membranes used and the operating conditions are the same as described in the preceding paragraph. The DF treatment was terminated when the weight of the retentate (composition containing GMP and LA) reached 30kg.

100kg of the liquid (composition containing GMP and LA) in combination with 70kg of the permeate solution obtained in the previous paragraph and 30kg of the re-permeate solution obtained in the previous paragraph was concentrated by a cross-flow method using a filtration film (polyether sulfone material, fraction molecular weight 5000 Da, membrane area 0.33 m²). The treatment conditions were 10°C in temperature and 0. 4MPa in mean operating pressure. This treatment gave 5kg of concentrate (a composition containing GMP and LA), and 95kg of permeate.

Subsequently, 5kg of the concentrate containing GMP and LA obtained in the preceding paragraph was subjected to DF treatment. The filtration membranes used and the operating conditions are the same as in the previous paragraph. The DF treatment was terminated when the weight of the permeate reached 30kg.

After the DF treatment, 5kg of the concentrate containing GMP and LA was freeze-dried to obtain 94g of a composition containing GMP and LA in powder form.

The content of GMP in dry powder form was 35% by mass per solid content, and the content of LA was 58% by mass. The content of β-lactoglobulin was 7% by mass. The recovery of GMP from the raw material used was 21%.

## Claims

1. A method for producing a composition containing κ-casein glycomacropeptide containing:
(A) preparing an aqueous solution containing a milk ingredient having a pH of 4 to 9;
(B) pretreating the aqueous solution to obtain an pretreated aqueous solution containing carbonate (hydrogen) ions and metal ions; and
(C) subjecting the pretreated aqueous solution to membrane treatment using a membrane having a molecular weight fraction of 9,000Da or more and 300,000Da or less.

2. The method for producing the composition according to claim 1, wherein the composition containing κ-casein glycomacropeptide is obtained as the permeate of the step (C) .

3. The method for producing the composition according to claim 1 or claim 2, wherein the method comprises adjusting pH of an aqueous solution containing a milk raw material having pH of less than 4 to pH of 4 or more prior to the step (C).

4. The method for producing the composition according to any one of claims 1 to 3, further containing (D) subjecting the concentrate obtained in the step (C) to the membrane treatment again to obtain a re-permeate obtained by the treatment.

5. The method for producing the composition according to any one of claims 1 to 4, further containing (E) concentrating the permeate and/or re-permeate using a membrane having a molecular weight fraction of less than 9,000Da.

6. The method for producing the composition according to any one of claims 1 to 5, wherein the fractional molecular weight of the membrane used in the step (C) is 30,000Da to 100,000Da.
